# EUROPEAN PATENT SPECIFICATION

(11) **EP 0 830 608 B1**
(45) Date of publication and mention of the grant of the patent: **04.10.2000**
(21) Application number: 96919107.1
(22) Date of filing: 06.06.1996
(51) Int. Cl.: G01N 33/86, C12Q 1/56

(54) **THROMBOSIS RISK TEST**
TEST FÜR THROMBOSEGEFAHR
TEST POUR DETERMINER LE RISQUE DE THROMBOSE

(30) Priority: 09.06.1995 US 488510
(43) Date of publication of application: 25.03.1998
(73) Proprietor: INSTRUMENTATION LABORATORY S.p.A., I-20128 Milano (IT)
(72) Inventor: CAMPBELL, Paula, A., Boston, MA 02110 (US); PREDA, Luigi, I-20050 Verano Brianza (IT)
(74) Representative: Perani, Aurelio
(86) International application number: PCT/US96/09036
(87) International publication number: WO 96/42018

(56) References cited:
- EP-A- 0 236 985
- EP-A- 0 406 971
- EP-A- 0 434 377
- EP-A- 0 445 626
- EP-A- 0 633 473
- WO-A-93/10261

## Description

### Background of the Invention

The present invention relates to an analytical global test to determine the risk of thrombosis in an individual by analysis of the Protein C/Protein S functionality in said individual.

Screening tests for coagulation disorders often are used to target patients at risk for bleeding. Such tests include, for example, the activated partial thromboplastin time (APTT) and the prothrombin time (PT) tests. Screening tests for coagulation disorders, or risk of bleeding, are designed to detect a significant abnormality in one or more of the clotting factors and to localize this abnormality to various steps in the coagulation pathway.

As commonly understood, coagulation may occur by two pathways, the intrinsic pathway and the extrinsic pathway. The former is generally triggered by the presence of a surface, and with the presence of phospholipids and calcium, through a number of steps eventually stimulates the formation of a stabilized fibrin clot. The APTT test typically measures coagulation factors of the intrinsic pathway, where most congenital deficiencies occur, and the PT test measures coagulation factors of the extrinsic pathway.

The APTT test typically is performed by adding an activator such as kaolin, silica, ellagic acid, and the like with phospholipid based reagents, to plasma. This activates Factors XII and XI. Currently, in the APTT, the phospholipids employed are extracted from bovine or rabbit brain, although sources such as soy bean have been used. The exogenous phospholipids of the APTT reagent substitute for the phospholipids provided by platelets in vivo in the activation of Factor X by Factors IX, VIII and V. Blood coagulation is limited in this clotting test by adding calcium. Factor VII is the only factor not affected by partial thromboplastin time. Thus, the APTT is normal in patients with a Factor VII deficiency.

The PT test typically is performed by adding tissue thromboplastin with calcium to plasma. This initiates clotting by activating Factor VII, which in turn activates Factor X which, in the presence of Factor V, leads to the conversion of prothrombin to thrombin. The thrombin which is so produced converts fibrinogen to fibrin. The PT test therefore bypasses the intrinsic clotting pathway and is normal in patients with deficiencies of Factors XII, XI, IX, and VIII. PT is abnormal in patients with deficiencies of Factors VII, X, V, prothrombin or fibrinogen. Tissue thromboplastin is a phospholipid extract (e.g., from rabbit brain or lung or human brain or placenta) to which calcium has been added.

Protein C and Protein S (PC and PS, respectively) also are involved in the coagulation process. Protein C is a double-chained, vitamin K-dependent glycoprotein in plasma which is synthesized in the liver. A physiologically indifferent coagulation precursor (decarboxyprotein C) is thereby first formed. Carboxylation of γ-glutamic acid residues in the protein by a vitamin K-dependent carboxylate results in the formation of protein C. Protein C itself is a pro-enzyme and is converted by thrombin into activated protein C (aPC). The latter acts as an anti-coagulant by a proteolytic inactivation of the activated coagulation Factors V and VIII. The inhibitory action of the active Protein C is increased by a cofactor, Protein S. Protein S is a single-chained glycoprotein in plasma which is also vitamin K-dependent. Active protein C and protein S form an equimolar complex. A lowered protein C level, as well as protein S level, have been described in patients with liver diseases, disseminated intravascular coagulation (DIC) and after oral anticoagulant therapy. A congenital deficiency of protein C or of protein S results in an increased venous thromboembolic risk. Therefore, protein C, as well as protein S, play an important part not only in the case of physiological haemostasis, but also in many diseases, and, especially in the case of thrombosis.

In commercially available test processes, the amount of protein C in plasma is determined by enzyme-labelled antibodies. However, this process suffers from the disadvantage that the antibodies used for the determination also react with the above mentioned decarboxyprotein C. Since the plasma concentration of decarboxyprotein C frequently increases very considerably during the course of a treatment with anticoagulants, this process involves a large source of error which, under certain circumstances, can result in false or insufficient therapy.

For example, in some photometric methods, Protein C is activated using Protac® (available from Pentapharm, Switzerland), which is obtained from the venom of the snake *Agkistrodon contortrix contortrix,* together with a chromogenic protein C substrate. In this process, the preparation of the sample can be omitted, however, it is not possible to differentiate between carboxylated and non-carboxylated protein C. It is known that only carboxylated protein C is effective *in vivo.* Therefore, no information about the biological activity of the protein C molecule can be obtained with this process.

The amount of protein S in a plasma can be determined by an immuno-radiometric test, such as that described, for example, by Bertina et al (Thromb Haemostas., 52 (2), 268-272 (1985). This process, however, suffers from the disadvantage that the antibodies used for the determination also react with nonfunctional protein S, for example with decarboxyprotein S or with protein S complexed with C₄-binding protein. Under certain circumstances, this results in falsely positive protein S values. The authors also describe a potential method for determining protein S in plasma in which activated protein C is admixed with human plasma and the partial thromboplastin time (PTT) is determined. The length of the PTT is indicative of the concentration of functional protein S. The reaction can be identified by the cleavage of fibrinogen by thrombin and the formation of a fibrin clot. However, the detection processes are limited, protein C and protein S cannot be simultaneously determined, and purified activated protein C must be used.

WO 93/10261 discloses a method in vitro for the diagnosis of blood coagulation disorders, in particular thromboembolic diseases, in a human or for the determination of the risk for a human to acquire blood coagulation disorders, wherein said disorders are not expressed by Protein S deficiency or defective Factor VIIIa, characterized in that the coagulation system in a sample is activated wholly or partly in a manner known per se and incubated with activated Protein C, whereupon a substrate conversion reaction rate like clotting or conversion of a chromogenic substrate is determined. The conversion rate obtained is compared with values obtained for normal plasma samples. If the rate is enhanced, it indicates that the individual from which the sample is derived may suffer from a thromboembolic disease.

EP-A- 0406971 discloses a method for determining the activity of protein S in a human plasma sample comprising combining the plasma sample with activated substrate plasma, then combining the mixture with bovine thromboplastin to initiate a coagulation reaction and measuring a parameter related to the time within which the coagulation reaction occurs.

Thus, there is a need for a rapid, global test for determining the thrombotic risk in a subject by surveying the activity of all factors, known or unknown on the biochemical mechanisms of coagulation.

### Summary of the Invention

The present invention is directed to an analytical global test for determining the thrombotic risk of a subject by the exploration of protein C/protein S functionality. Additional features and advantages of the invention will be set forth in the description which follows, and in part will be apparent from the description, or may be learned by practice of the invention. The objectives and other advantages of the invention will be realized and attained by the methods and kits particularly pointed out in the written description and claims hereof.

To achieve these and other advantages, and in accordance with the purpose of the invention, as embodied and broadly described herein, the invention provides a method for determining thrombotic risk in a subject by adding to a plasma sample obtained from the subject (i) a first reagent in an amount sufficient to induce or activate coagulation in the plasma, (ii) a second reagent which activates endogenous protein C in the plasma, and (iii) a third reagent comprising calcium salts, phospholipids or tissue thromboplastin, or a combination thereof.

One then adds to a second plasma sample from the same subject (i) a reagent which induces or activates coagulation, (ii) a buffer or other material which does not activate protein C, and (iii) a third reagent comprising calcium salts, phospholipids or tissue thromboplastin, or a combination thereof. The time, rate, or both, necessary for the conversion of endogenous fibrinogen to fibrin in both the first and second samples is measured. The same steps are performed on normal control plasma, and the difference or ratio in the times, rates, or both, obtained above are determined.

In other embodiments, the methods of the invention comprise adding to a plasma sample obtained from the subject a synthetic substrate, and a second reagent which activates protein C in the plasma; and to a second sample from the same subject adding a synthetic substrate and a second reagent which does not activate protein C in the plasma. The rates of hydrolysis of the synthetic substrates in each sample are then measured, and compared as above. The same steps are performed on normal control plasma, and the rates of hydrolysis are determined.

In some embodiments, the first reagent is selected from the group consisting of, but not limited to, Factor IXa, reagents which generate Factor IXa *in vitro,* and APTT reagents. In other embodiments, the first reagent is selected from the group consisting of Factor Xa, reagents which generate Factor Xa *in vitro,* PT reagents, and activators of the extrinsic coagulation pathway.

Preferred second reagents may be a purified fraction of venom obtained from a venom producing organism, such as an arachnid or a reptile, i.e., snake or spider venom. In the currently preferred embodiment, the second reagent is a snake venom fraction obtained from *Agkistrodon contortrix contortix.* In other embodiments, the second reagent may be a recombinant venom protein or polypeptide.

A kit for determining the thrombotic risk in a subject also is the subject of the present invention. In one embodiment, the kit comprises a first container which has (i) a first reagent in an amount sufficient to induce or activate coagulation in a plasma sample obtained from the subject, (ii) a second reagent which activates endogenous protein C in the plasma, and (iii) a third reagent comprising calcium salts, phospholipids or tissue thromboplastin, or a combination thereof.

The kit of the invention further comprises a second container for adding to a second plasma sample from the same subject a first reagent which activates or induces coagulation; a buffer or other material which does not activate protein C; and calcium salts, phospholipids or tissue thromboplastin, or a combination thereof.

In some embodiments, the kit comprises at least one container having therein a first reagent comprising a synthetic substrate, and a second reagent which activates protein C; and at least one second container having therein a first reagent comprising a synthetic substrate, and a second reagent comprising a buffer or other material which does not activate protein C. It is understood that the first and second reagents may be combined in a single container (e.g., a vial) or may be provided in separate containers.

The kit may contain first reagents selected from the group consisting of Factor IXa, reagents which generate Factor IXa *in vitro* and APTT reagents. Alternatively, the first reagent may be selected from the group consisting of Factor Xa, reagents which generate factor Xa *in vitro,* PT reagents, and activators of the extrinsic coagulation pathway.

In preferred embodiments, the second reagent comprises a purified fraction of venom obtained from a venom producing organism, such as an arachnid or snake. In a currently preferred embodiment, the second reagent is a purified snake venom fraction obtained from *Agkistrodon controtrix contortrix.* The second reagent also may be a recombinant venom protein or polypeptide.

It is to be understood that both the foregoing general description and the following detailed description are exemplary and explanatory and are intended to provide further explanation of the invention as claimed.

The accompanying drawings are included to provide a further understanding of the invention and are incorporated in and constitute a part of this specification, illustrate one embodiment of the invention and together with the description serves to explain the principles of the invention.

### Brief Description of the Drawings

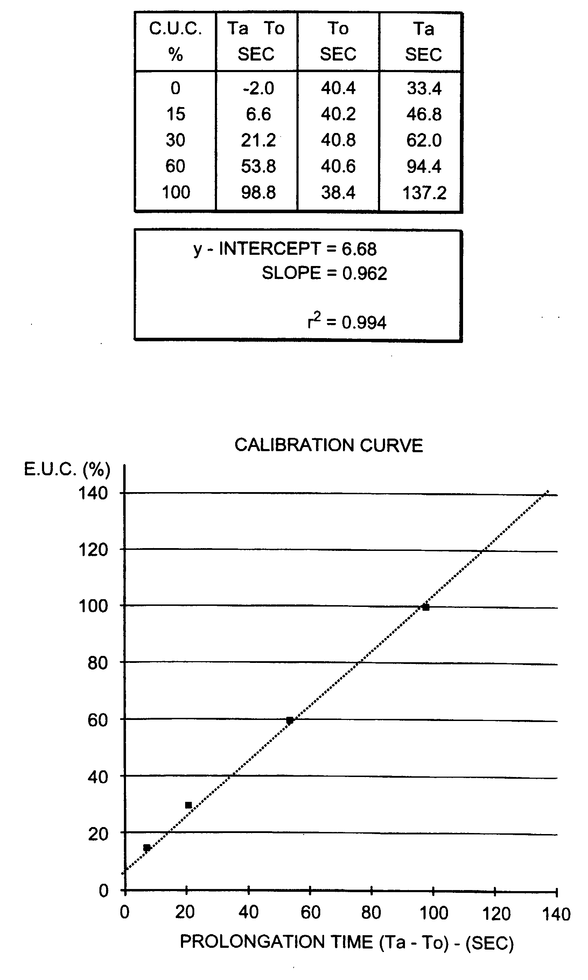

The Figure is a calibration curve for the calculation of the coagulative activity, as measured in Equivalent Units of Protein C (EUC), assuming that normal plasma has an activity of 100 % EUC, and a plasma deficient in protein C has an activity of 0 % EUC.

### Detailed Description of the Invention

The present invention relates to an analytical global test useful for the exploration of protein C/protein S functionality. The global test can be a diagnostic aid for the evaluation of thrombotic risk in patients suffering from hereditary and non-hereditary thrombophilia (i.e., disorders of the hemopoietic systems in which there is a tendency toward the occurrence of thrombosis), or otherwise in patients undergoing particularly pharmacologic treatments such as extroprogestinics, etc. Additionally, patients undergoing surgery can be evaluated to determine thrombotic risk.

The claimed invention is an analytical *in vitro* test that can be used to diagnose clotting anomalies involved in the inhibitory system, due to either PC deficiency, PS deficiency, or to the presence of molecular anomalies of Factor Va, or to the presence of anti-aPC antibodies. Generally, the methods of the invention provide that patient plasma is incubated with an exogenous reagent able to activate or induce clotting activity; an exogenous reagent that transforms endogenous PC to aPC, and other components such as calcium salts, phospholipids or tissue thromboplastin necessary to complete the clotting reaction. To a second sample of plasma from the same patient is added the first reagent, the third reagent, and in lieu of the second reagent, a substance which does not activate PC, thus showing the patient's global clotting activity. The same steps are repeated using a normal plasma control sample.

The times or rates necessary for the conversion of fibrinogen into fibrin are measured for both samples, and the ratio or difference between the non-activated sample and the activated sample is determined. The ratio or difference is indicative of the presence of potential thrombotic risk. As an example, if the difference in time of the patient plasma sample is shorter than the normal control sample, then there is a high risk of a thrombotic event. The methods and kits of the invention are discussed in further detail below.

The analytical test provides a method for determining the ratio between the clotting activity and the inhibitory activity of the protein C/ protein S system in a plasma sample. In one embodiment, the test involves first adding to a plasma sample from the subject a first reagent sufficient to induce or activate coagulation in the plasma. Any reagent capable of inducing or activating coagulation can be used. Preferred first reagents include, but are not limited to, Factor IXa, reagents which generate Factor IXa *in vitro,* APTT reagents, Factor Xa, reagents which generate Factor Xa *in vitro,* PT reagents and activators of the extrinsic coagulation pathway.

Activators of the extrinsic coagulation pathway may be any activators having the desired activity. Specifically, activators useful in the invention may include natural sourced or recombinant tissue thromboplastins, including, for example, bovine thromboplastin, human thromboplastin, rabbit thromboplastin and porcine thromboplastin. Bovine thromboplastin currently is preferred. Portions of such activators may also have the desired activity. A recombinant or synthetic mutant or modified thromboplastin having the desired activity also may be used for this purpose. Mutant or modified thromboplastins are recombinant or synthetic proteins in which a portion of the nucleotide sequence encoding the protein or the amino acid sequence is missing or altered, but which retains thromboplastin activity. One skilled in the art may readily identify other activators of the extrinsic coagulation pathway which would be useful in the claimed invention.

A second reagent also is added to the plasma sample obtained from the subject. The second reagent activates endogenous Protein C in the plasma. The second reagent can be any substance having the desired activity. For example, one may obtain the second reagent from the venom of venom producing organisms such as arachnids or reptiles. For example, snake venoms from which the desired fraction can be purified may be obtained from *Agistrodon contortrix contortrix, A.C. mokasen, A.C. pictigaster, A. piscivours, A. ieucostoma, A bilineatus, Bothrops moojeni, B. pradoi, Cerastes cerastes, Vipera lebetrina or V*. *russelli.* Preferably, the second reagent is snake venom, most preferably a fraction obtained from *Agkistrodon contortrix contortrix.* Such a reagent is commercially available under the tradename Protac.® The second reagent may, in some instances, be a derivatized or modified form of the venom, or in other embodiments at least a portion of a recombinant venom protein or polypeptide having the desired activity.

The third reagent added to the plasma sample may comprise calcium salts, phospholipids or tissue thromboplastin, or any combination thereof.

A second sample obtained from the same subject is combined with the first reagent which activates coagulation, a buffer or other material which does not activate Protein C, and the third reagent, e.g., calcium salts, phospholipids, or tissue thromboplastin or any combination thereof. One then measures the conversion of endogenous fibrinogen to fibrin in both the first sample and the second sample. The measurements may, for example, determine the rate of conversion, the time of conversion, or both. One skilled in the art can easily select which measurements best suits each individual application. If the third reagent is a synthetic substrate, the hydrolysis of the substrate is measured instead of the conversion of fibrinogen to fibrin.

The rate or time of conversion of fibrinogen to fibrin can be measured by methods known in the art for determining coagulation times or rates, such as, for example, by measuring the change in optical density of the sample.

The difference or ratio between the measurements of the rate or time (or both) obtained above is then calculated, and the identical steps performed on normal control plasma. Then, one determines the difference or ratio 1) in the times, rates (or both) obtained for the normal control; and 2) the first and second samples. The difference is indicative of the thrombotic risk in the subject. If the time, rate, or both, calculated for the test plasma is shorter than the time, rate or both for the normal control plasma, the patient is at increased thrombotic risk.

Specifically, shorter conversion times or rates are indicative of a deficiency in Protein C, Protein S, or both. Lowered or reduced levels of Protein C or Protein S may result in an increased risk of clot formation. Alternatively, a high level of PC or PS will indicate a normal coagulation pattern, and thus, a low risk of thrombosis.

In other embodiments, the methods of the invention comprise adding to a first sample from the subject a synthetic substrate, and a second reagent which activates protein C. The second sample from the subject is combined with a synthetic substrate and a second reagent which does not activate protein C. In these embodiments, the rate of hydrolysis of the synthetic substrate in the first and second samples are measured and compared.

The synthetic substrate, may, for example be a thrombin substrate, useful for monitoring the reaction. Thrombin substrates may include synthetic fibrinogen or portion thereof, or other protein or polypeptide which can be converted by thrombin to a detectable substance. The synthetic substrate may comprise a label or tag such that the rate of hydrolysis of the substrate may be measured. It is contemplated that the label or tag be intrinsic to the substrate, or a detectable moiety attached to the substrate.

The methods of the invention may be carried out manually, or automatically. The methods preferably are carried out automatically using a specially designed instrument, such as those available from Instrumentation Laboratory, S.p.A., Milan, Italy. The samples are loaded into the instrument, and reagents are added, and the conversion times or rates are determined in accordance with the manufacturer's instructions.

The claimed invention also encompasses kits for determining thrombotic risk in a subject. The kits contain a first container having (i) a first reagent in an amount sufficient to induce or activate coagulation in a plasma sample obtained from the subject, (ii) a second reagent which activates endogenous Protein C in the plasma, and (iii) a third reagent comprising calcium salts, phospholipids or tissue thromboplastin, or a combination thereof. The term "container" as used herein means any container capable of housing the reagents listed above. In various embodiments, the first container may comprise a separate vial for each reagent, a single vial with all three reagents, or, two vials containing any combination of the three reagents. One skilled in the art may choose any configuration for the first container, based upon the particular application and convenience of use. The container may optionally be a disposable, single use container, or, in other embodiments, may be a container capable of sterilization and reuse.

The kit of the invention also has a second container for adding to a second plasma sample from the same subject (i) a first reagent, (ii) a buffer or other material which does not activate Protein C, and (iii) a third reagent comprising calcium salts, phospholipids or tissue thromboplastin, or a combination thereof.

The reagents used in the kits may be the same reagents as discussed above in relation to the methods of the invention.

In some embodiments, the kits of the invention have a first container comprising a synthetic substrate and a second reagent which activates protein C. The second container comprises a synthetic substrate and a buffer or material which does not activate protein C. In other embodiments the kits of the invention may contain only one container, i.e., either the first or the second container.

Thus, as described above, the claimed invention allows the rapid evaluation of the functionality of PC and PS in subjects. The tests of the invention can be a valid diagnostic aid for screening patient samples to recognize those patients with high thrombotic risk, i.e., hereditary PC deficiency, heterozygous or homozygous mutations, such as F.V. Leiden mutation, and patients undergoing surgery. The methods of the invention encompass a global test because they are capable of detecting the influence of other factors, known or unknown, in addition to PC and PS, on the biochemical mechanism that allows effective activated PC inhibiting action on the physiological substrates (i.e., Factors Va and VIlla).

The global activity of the system may be measured in equivalent units of protein C, arbitrarily assigning an activity value of 100% to a pool of normal patient plasma and 0% to a PC deficient plasma. Other means of measurement may be used to obtain the relative activity of the PC/PS system, and are easily determined by the skilled artisan.

### EXAMPLES

### Example 1

The analytical test below can be carried out either manually or automatically. The volumes reported below were obtained using the Research Program of the ACL 3000/p system (available from Instrumentation Laboratory, S.p.A., Milan, Italy).

50 µL of undiluted patient plasma were separately loaded in a rotor with 50 µL of H₂O (or similar buffer), and centrifuged. After 150 seconds, 50 µL of calcium bovine thromboplastin was added. The coagulation reaction was allowed to proceed for 240 seconds, and the coagulation time was calculated using the principles of the ACL. The measured coagulation time, or rate (To) represents the coagulative activity of the plasma sample.

In a second rotor, another sample from the same subject was processed as above, except that the H₂O is replaced with a PROTAC™ solution ( fractionated *Agkistrodon contortrix contortrix* venom, 0.125 U/mL conc.). The coagulation time, or rate (Ta) was then measured.

The ratio of (Ta/To) or the difference (Ta-To) represents an index of the total functionality of the PC-PS inhibitor system.

As an example, the values given below were obtained by the method described in the example above, and represent the To, Ta, and % E.U.C. of patients with a deficiency of PC, PS or with an increased aPC resistance. The % E.U.C. for normal plasma is between 57 and 153.

| Defect | To | Ta | % E.U.C. |
|---|---|---|---|
| PC deficiency | 45.6 | 84.0 | 43 |
| | 39.4 | 66.0 | 39 |
| PS deficiency | 49.2 | 50.0 | 1 |
| | 49.0 | 58.0 | 13 |
| aPC resistance | 32.8 | 37.2 | 4 |
| | 30.2 | 34.2 | 12 |

### Example 2

### MATERIALS AND METHODS

### Collection of Blood Samples

Blood samples (20 ml) were collected by venipuncture with 21-gauge butterfly infusion sets into plastic syringes containing 3.8% (wt/vol) sodium citrate in a ratio of 0.1:0.9 (vol/vol) anticoagulant to blood. Platelet poor plasma was obtained by centrifugation at 2000 x g for 20 minutes and stored at -80°C until it was analyzed.

### Routine coagulation assays

Laboratory investigations including the measurement of activated-partial thromboplastin time (aPTT) and prothrombin time (PT), assays for antithrombin III and plasminogen, PC antigen and activity, total and free PS antigen and PS activity, have been performed as previously described. Girolami et al., Br. J. Haematol. 85:521-526 (1993); Girolami et al., Thromb. Haemost. 61:144-147 (1989); Preda et al., Thomb. Res., 60:19-32 (1990).

A reference pool of normal plasma for all these assays was obtained from 40 healthy subjects of both sexes, aged 20 to 60 years. Normal values for each test were determined in 80 healthy individuals of both sexes, aged 20 to 70 years.

The criteria used for the classification of AT III, PC and PS defects were in accordance with those reported in the current literature. Dahlback, Thromb. Haemost., 74:139-148 (1995); Alach et al., Thromb. Haemost., 74:81-89 (1995).

Evaluation of the response to APC in plasma (APC resistance test). The test was performed on an ACL 3000plus/R (Instrumentation Laboratory, S.p.A., Milan, Italy) using a modification of the methods proposed by Bertina et al. (Nature, 369:64-67 (1994); Thromb. Haemost., 72:880-886 (1994)). Briefly, fifty microliters of undiluted plasma were incubated with 50 ul of aPTT-lyophilized silica (Instrumentation Laboratory, S.p.A., Milan, Italy) for 300 seconds at 37°C. Clot formation was started either by addition of 50 ul of 25 mM calcium-chloride, 25 mM Tris-HCI (pH 7.5), 50 mM NaCI and 0.05% ovalbumin (aPTT-APC) or by 50 ul of the same reagent containing also 1.0 ug/ml (final concentration) of human APC (Enzyme Research Laboratory, South Bend, Indiana, USA) (aPTT+APC). An "APC sensitivity ratio" (APC-SR) was calculated dividing the aPTT+APC (seconds) by the aPTT-APC (seconds). The APC-SR was then normalized (n-APC-SR) to the ratio obtained with a reference plasma. Resistance to APC is defined by n-APC-SR less than 0.80. A n-APC-SR between 0.45 and 0.70 was consistent with the presence of heterozygous F.V Leiden mutation whereas the diagnosis of homozygous defect was made if the n-APC-SR was < 0.45.

DNA analysis for F.V Leiden mutation. High molecular weight DNA was extracted from 5 µl of peripheral blood using a binding matrix (Biorad Lab, Hercules, CA, USA). DNA analysis was performed as previously described. Bertina et al., Nature, 369:64-67 (1994); Simoni et al., Stroke, 28:885-890 (1995).

### PC-PS system global test

This test consists of the measurement of the PT of a plasma sample before the activation (PTo) and after the activation of the native PC (PTa) present in the sample by PROTAC®, a snake venom derived from *Agkistrodon Contortrix Contortrix* obtained from Pentapharm (Basel, Switzerland).

The prolongation of the clotting time (PTa-PTo) after activation of the native PC reflects the global activity of the PC-PS system. The global activity of the PC-PS system is expressed as the percentage of Equivalent Unit of PC (EUC%). Therefore PTa-PTo obtained in a pooled normal plasma corresponded to 100 EUC whereas that obtained in PC deficient plasma was consistent with 0 EUC. A calibration curve was obtained by diluting pooled normal plasma (PNP) in PC deficient plasma.

The test was carried out in a two step cycle, using the ACL 3000plus/R coagulometer, at the following analytical conditions:

| | |
|---|---|
| sample | 50 µl |
| reagent 1 | 50 µl |
| reagent 2 | 50 µl |
| | |
| activation time | 150 sec |
| inter-ramp interval | 3 sec |
| delay time | 10 sec |
| acquisition time | 240 sec |
| speed | 1200 rpm |

Clotting time was calculated using the following elaboration path:

| | |
|---|---|
| Mode | S/Rx100 |
| Data | Sml=15, Sm2=11 |
| First derivative | Calc=3, Sm1=11 |
| Criterion | 1 st derivative |

As mentioned above, the PC-PS system global test consists of a double PT. Reagent 1 was H₂O and PROTAC® at a final concentration of 0.125 U/ml, in the basal PT and in PT performed after activation of native PC, respectively. Reagent 2 was a bovine thromboplastin (Instrumentation Laboratory, S.p.A., Milan, Italy), containing 20 mM Ca-ion and polybrane as a heparin inhibitor.

EUC% was calculated as follows:$\text{EUC% =} \frac{{\text{(PTa - PTo)}}_{\text{sample}}}{{\text{(PTa - PTo)}}_{\text{NP}}} \text{x 100}$

As normal plasma we used a dried plasma (Calibration plasma, lot #N0743187, (Instrumentation Laboratory, S.p.A., Milan, Italy) in which the activities of all clotting factors were > 90%, while PC and PS functional activities were 90% and 98% respectively.

Statistical analysis. Differences between the groups were evaluated by a non-parametric test (unpaired Wilcoxon test) and p values > 0.05 were not considered significant (NS).

### RESULTS

The evaluation of PC/PS system activity was performed in 231 plasma samples. One hundred and twelve belonged to normal individuals (control group), 36 to patients with PC deficiency, 22 with PS deficiency, 35 with aPC resistance due to F.V Leiden mutation and 26 with thrombophilia due to other coagulation disorders (2 with ATIII deficiency, 1 with HC II deficiency, 1 with plasminogen deficiency and 12 other with hyperhomocystinemia). Table 1 shows the dose-response curve for the test. When the prolongation of the clotting time (Ta-To) was plotted against the percentage of EUC, a linear correlation was obtained.

**Table 1**

| **DOSE-RESPONSE CURVE** | | | |
|---|---|---|---|
| **EUC** | **To** | **Ta** | **Ta-To** |
| **%** | **sec** | **sec** | **sec** |
| 100 | 40,4 | 134,7 | 105,0 |
| 60 | 41,0 | 96,5 | 62,9 |
| 40 | 42,3 | 77,5 | 40,4 |
| 20 | 42,4 | 58,0 | 18,2 |
| 10 | 42,4 | 49,0 | 8,0 |
| 0 | 42,7 | 40,7 | -1,0 |

Table 2 shows the data of the intra and inter-assay coefficient of variation (CV%) of the global test, determined for three levels of EUC% corresponding to a calibration plasma (NP #NO743187), a control plasma containing 40% PS and a control plasma containing 20% PC. The assay precision gave satisfactory results.

**Table 2**

| | **NP #NO743187** | **PS control 40%** | **PC control 20%** |
|---|---|---|---|
| EUC % average | 103,0 | 13,1 1 | 15,2 |
| CV% intra- | 1,5-6,5 | 1,3-6,8 | 1,4-7,5 |
| inter- | 4,6 | 6,5 | 6,7 |

Table 3 contains the data of the control group as well as those of four patient groups. Shown are the number of samples according to their deficiency status, mean EUC% values, median EUC% values, number of samples for which the EUC% levels were in agreement with the deficiency status.

**Table 3**

| | **N°** | **EUC %** | | **N°** |
|---|---|---|---|---|
| | | **average ± 1 std** | **median** | |
| Normal control | 112 | 96,0 ± 25,3 | 93,5 | 103 |
| PC defect | 36 | 50,8 ± 18,2 | 41,0 | 33 |
| PS defect | 22 | 32,4 ± 28,7 | 24,5 | 20 |
| aPC-resistant | 35 | 26,2 ± 13,9 | 25,0 | 34 |
| Other | 26 | 95,6 ± 31,9 | 83,5 | 26 |
| TOTAL | 231 | | | |

The present test showed a high sensitivity for the deficiency of the two principal inhibitors, PC (33/36, 91.7%) and PS (20/22, 90.9%) as well as for F.V Leiden mutation (34/35, 97.1%). Out of 112 individuals of the control group, 103 (92%) were identified as normal by the global activity test. All other patients with defects different from those of the PC/PS system had a normal global test (26/26, 100%).

In the PC/PS global test unfractionated heparin was tolerated up to a concentration of 0.4 U/ml.

Genetic defects of PC, PS, AT considered together, are found in only 5-10% of patients presenting with familial venous thrombophilia. In about 90% of cases, APC-resistance is caused by a single point mutation in the F.V gene predicting the replacement of Arg506 with Gln. Bertina et al., Nature, 369:64-67 (1994). Depending on the selection of patients taken into account, F.V Leiden was found in about 20-50% of cases of thrombophilia. Defects involving the PC/PS system are nowadays the most frequent cause of inherited thrombophilia. Impairment of the PC/PS system due to the presence of antiphospholipid antibodies is maintained to be a pathogenetic mechanism of thombosis in some cases.

As shown by the results obtained above, the sensitivity of the test of the invention in the detection of PC and PS defects was high. Only three patients out of 36 with PC deficiency went undetected, 2 out of 22 with PS deficiency and only 1 out of 35 with F.V. Leiden mutation. Nine patients belonging to the control group exhibited an abnormal response to the PC/PS global test. No PC or PS defects or APC resistance was found in these individuals. The presence of antiphospholipid antibodies also was excluded. None of the other 26 patients with thrombophilia due to other genetic defects showed abnormal PC/PS global activity. Native PC in the PC/PS global activity test is activated by PROTAC®, therefore, it is likely that impairment of physiological activation of PC by thrombin-thrombomodulin complex cannot be detected by this assay.

The present method may be usefully employed as a screening-test in the daily screening of subjects with increased thrombotic risk related to the reduction of the global activity of the PC-PS system.

## Claims

1. A method for determining thrombotic risk in a subject, said method comprising:
a. adding to a plasma sample obtained from said subject (i) a first reagent in an amount sufficient to induce or activate coagulation in the plasma, (ii) a second reagent which activates endogenous Protein C in the plasma, and (iii) a third reagent comprising calcium salts, phopholipids or tissue thromboplastin, or a combination thereof;
b. adding to a second plasma sample from the same said subject (i) a first reagent which activates or induces coagulation, (ii) a buffer or other material which does not activate protein C, and (iii) a third reagent comprising calcium salts, phospholipids or tissue thromboplastin, or a combination thereof;
c. measuring the time, rate, or both, necessary for conversion of endogenous fibrinogen to fibrin in the sample of step (a);
d. measuring the time, rate, or both necessary for conversion of endogenous fibrinogen to fibrin in the plasma sample of step (b);
e. calculating the difference or ratio between the times, rates or both, obtained in steps (c) or (d);
f. performing steps (a), (b), (c) and (d) on a sample of normal control plasma; and
g. determining the difference or ratio in the times, rates, or both, obtained in steps (e) and (f) wherein said difference is indicative of the thrombotic risk in said subject.

2. A method for determining thrombotic risk in a subject, said method comprising
a) adding to a plasma sample obtained from said subject (i) a first reagent comprising a synthetic substrate, and (ii) a second reagent which activates endogenous Protein C in the plasma;
b) adding to a second plasma sample from the same said subject (i) a first reagent comprising a synthetic substrate, and (ii) a buffer or other material which does not activate Protein C;
c) measuring the rate of hydrolysis of the synthetic substrate in the sample of step (a);
d) measuring the rate of hydrolysis of the synthetic substrate in the sample of step (b);
e) calculating the difference between the rates obtained in steps (c) and (d);
f) performing steps (a), (b), (c), and (d) on a sample of normal control plasma; and
g) determining the difference or ratio in the rates obtained in steps (e) and (f) wherein said difference is indicative of the thrombotic risk in said subject

3. The method of claim 1 wherein said first reagent is selected from the group consisting of Factor IXa, reagents which generate Factor IXa *in_vitro* and APTT reagent.

4. The method of claim 1 wherein the first reagent is selected from the group consisting of Factor Xa, reagents which generate Factor Xa *in vitro,* PT reagents, and activators of the extrinsic coagulation pathway.

5. The method of claim 1 or 2 wherein said second reagent comprises a purified fraction of venom obtained from a venom producing organism

6. The method of claim 5 wherein the venom is snake venom obtained from *Agkistrodon contortrix contortrix.*

7. The method of claim 1 or 2 wherein the second reagent comprises at least a portion of a recombinant venom protein.

8. A kit for determining thrombotic risk in a subject, said kit comprising:
a. a first container comprising: (i) a first reagent in an amount sufficient to induce or activate coagulation in a plasma sample obtained from said subject, (ii) a second reagent which activates endogenous Protein C in the plasma, and (iii) a third reagent comprising calcium salts, phospholipids or tissue thromboplastin, or a combination thereof;
b. a second container for adding to a second plasma sample from said same subject (i) said first reagent, (ii) a buffer or other material which does not activate Protein C, (iii) a third reagent comprising calcium salts, phospholipids or tissue thromboplastin, or a combination thereof.

9. A kit for determining thrombotic risk in a subject, said kit comprising:
a) a first container comprising (i) a first reagent comprising a synthetic substrate, and (ii) a second reagent which activates endogenous Protein C in the plasma;
b) a second container for adding to a second plasma sample from the same said subject (i) said first reagent comprising a synthetic substrate, and, (ii) a buffer or other material which does not activate Protein C

10. The kit of claim 8 wherein said first reagent is selected from the group consisting of Factor IXa, reagents which generate Factor IXa *in vitro* and APTT reagents.

11. The kit of claim 8 wherein the first reagent is selected from the group consisting of Factor Xa, reagents which generate Factor Xa *in vitro,* PT reagents and activators of the extrinsic coagulation pathway.

12. The kit of claim 8 or 9 wherein said second reagent comprises a purified fraction of venom obtained from a venom producing organism.

13. The kit of claim 12 wherein the venom is snake venom obtained from *Agkistrodon contortrix contortrix.*

14. The kit of claim 8 or 9 wherein the second reagent comprises a recombinant venom protein.

## Patentansprüche

1. Verfahren zur Bestimmung des Thromboserisikos bei einem Patienten durch:
(a) Zugabe - zu einer von dem betreffenden Patienten gewonnenen Plasmaprobe -
(i) eines ersten Reagenses in einer zur Einleitung oder Aktivierung einer Koagulation in dem Plasma ausreichenden Menge;
(ii) eines zweiten Reagenses, welches endogenes Protein C in dem Plasma aktiviert, und
(iii) eines Calciumsalze, Phospholipide oder Gewebethromboplastin oder eine Kombination hiervon umfassenden dritten Reagenses;
(b) Zugabe - zu einer zweiten von dem gleichen Patienten gewonnenen Plasmaprobe -
(i) eines eine Koagulation aktivierenden oder einleitenden ersten Reagenses;
(ii) eines Puffers oder eines sonstigen, Protein C nicht aktivierenden Materials, und
(iii) eines Calciumsalzes, Phospholipide oder Gewebethromboplastin oder eine Kombination hiervon umfassenden dritten Reagenses;
(c) Bestimmen der zur Umwandlung von endogenem Fibrinogen zu Fibrin in der Probe von Stufe (a) erforderlichen Zeit und/oder Geschwindigkeit;
(d) Bestimmen der zur Umwandlung von endogenem Fibrinogen zu Fibrin in der Probe von Stufe (b) erforderlichen Zeit und/oder Geschwindigkeit;
(e) Berechnen des Unterschieds oder Verhältnisses zwischen den in Stufen (c) oder (d) ermittelten Zeiten und/oder Geschwindigkeiten;
(f) Durchführen der Stufen (a), (b), (c) und (d) mit einer Probe normalen Kontrollplasmas, und
(g) Bestimmen des Unterschieds oder Verhältnisses der in den Stufen (e) und (f) ermittelten Zeiten und/oder Geschwindigkeiten, wobei der Unterschied ein Anzeichen für das Thromboserisiko bei dem betreffenden Patienten darstellt.

2. Verfahren zur Bestimmung des Thromboserisikos bei einem Patienten durch
(a) Zugabe - zu einer von dem betreffenden Patienten gewonnenen Plasmaprobe -
(i) eines ersten ein synthetisches Substrat umfassenden Reagenses und
(ii) eines endogenes Protein C in dem Plasma aktivierenden zweiten Reagenses;
(b) Zugabe - zu einer zweiten von dem gleichen Patienten gewonnen Plasmaprobe -
(i) eines ein synthetisches Substrat umfassenden ersten Reagenses und
(ii) eines Puffers oder eines sonstigen, Protein C nicht aktivierenden Materials;
(c) Messen der Hydrolysegeschwindigkeit des synthetischen Substrats in der Probe von Stufe (a);
(d) Messen der Hydrolysegeschwindigkeit des synthetishen Substrats in der Probe von Stufe (b);
(e) Berechnen des Unterschieds zwischen den in Stufen (c) und (d) ermittelten Geschwindigkeiten;
(f) Durchführen der Stufen (a), (b), (c) und (d) mit einer Probe eines normalen Kontrollplasmas, und
(g) Bestimmen des Unterschieds oder Verhältnisses der in den Stufen (e) und (f) ermittelten Geschwindigkeiten, wobei der betreffende Unterschied ein Anzeichen für das Thromboserisiko bei dem betreffenden Patienten darstellt.

3. Verfahren nach Anspruch 1, wobei das erste Reagens aus der Gruppe, bestehend aus Faktor IXa, Reagenzien, die in vitro Faktor IXa erzeugen, und APTT-Reagens, ausgewählt ist.

4. Verfahren nach Anspruch 1, wobei das erste Reagens aus der Gruppe, bestehend aus Faktor Xa, Reagenzien, die in vitro Faktor IXa erzeugen, PT-Reagenzien und Aktivatoren für den exogenen Koagulationspfad, ausgewählt ist.

5. Verfahren nach Anspruch 1 oder 2, wobei das zweite Reagens eine gereinigte Fraktion von aus einem Tiergift produzierenden Organismus gewonnenem Tiergift umfaßt.

6. Verfahren nach Anspruch 5, wobei das Tiergift aus von Agkistrodon contortrix contortrix gewonnenem Schlangengift besteht.

7. Verfahren nach Anspruch 1 oder 2, wobei das zweite Reagens mindestens einen Teil eines rekombinanten Tiergiftproteins umfaßt.

8. Kit zur Bestimmung des Thromboserisikos bei einem Patienten, umfassend:
(a) einen ersten Behälter mit
(i) einem ersten Reagens in einer zur Einleitung oder Aktivierung einer Koagulation in einer von dem betreffenden Patienten gewonnenen Plasmaprobe ausreichenden Menge,
(ii) einem endogenes Protein C im Plasma aktivierenden zweiten Reagens und
(iii) einem Calciumsalze, Phospholipide oder Gewebethromboplastin oder eine Kombination hiervon umfassenden dritten Reagens, und
(b) einen zweiten Behälter zum Zusatz
(i) des ersten Reagenses,
(ii) eines Puffers oder eines sonstigen, Protein C nicht aktivierenden Materials, und
(iii) eines Calciumsalze, Phospholipide oder Gewebethromboplastin oder eine Kombination hiervon umfassenden dritten Reagenses zu einer zweiten Plasmaprobe von dem gleichen Patienten.

9. Kit zur Bestimmung des Thromboserisikos bei einem Patienten, umfassend
(a) einen ersten Behälter mit
(i) einem ein synthetisches Substrat umfassenden ersten Reagens und
(ii) einem ein endogenes Protein C im Plasma aktivierenden zweiten Reagens, und
(b) einen zweiten Behälter zum Zusatz
(i) des ein synthetisches Substrat umfassenden ersten Reagenses und
(ii) eines Puffers oder eines sonstigen, Protein C nicht aktivierenden Materials zu einer zweiten von dem gleichen Patienten gewonnenen Plasmaprobe.

10. Kit nach Anspruch 8, wobei das erste Reagens aus der Gruppe, bestehend aus Faktor IXa, Reagenzien, die in vitro Faktor IXa erzeugen, und APTT-Reagenzien, ausgewählt ist.

11. Kit nach Anspruch 8, wobei das erste Reagens aus der Gruppe, bestehend aus Faktor Xa, Reagenzien, die in vitro Faktor Xa erzeugen, PT-Reagenzien und Aktivatoren für den exogenen Koagulationspfad ausgewählt ist.

12. Kit nach Anspruch 8 oder 9, wobei das zweite Reagens eine gereinigte Fraktion eines von einem Tiergift produzierenden Organismus gewonnenen Tiergifts umfaßt.

13. Kit nach Anspruch 12, wobei das Tiergift aus von Agkistrodon contortrix contortrix gewonnenem Schlangengit besteht.

14. Kit nach Anspruch 8 oder 9, wobei das zweite Reagens ein rekombinantes Tiergiftprotein umfaßt.

## Revendications

1. Méthode pour déterminer le risque de thrombose chez un sujet, ladite méthode comprenant les étapes consistant :
a. à ajouter à un échantillon de plasma obtenu chez ledit sujet (i) un premier réactif en une quantité suffisante pour induire ou activer la coagulation dans le plasma, (ii) un deuxième réactif qui active la protéine C endogène dans le plasma et (iii) un troisième réactif comprenant des sels de calcium, des phospholipides ou de la thromboplastine tissulaire ou bien une de leurs associations ;
b. à ajouter à un second échantillon de plasma provenant de ce même sujet (i) un premier réactif qui active ou induit la coagulation, (ii) un tampon ou une autre substance qui n'active pas la protéine C et (iii) un troisième réactif comprenant des sels de calcium, des phospholipides ou de la thromboplastine tissulaire ou bien une de leurs associations ;
c. à mesurer le temps, la vitesse ou ces deux paramètres nécessaires pour la conversion du fibrinogène endogène en fibrine dans l'échantillon de l'étape (a) ;
d. à mesurer le temps, la vitesse ou ces deux paramètres nécessaires pour la conversion du fibrinogène endogène en fibrine dans l'échantillon de plasma de l'étape (b) ;
e. à calculer la différence ou le rapport entre les temps, les vitesses ou ces deux paramètres obtenus dans les étapes (c) et (d) ;
f. à mettre en oeuvre les étapes (a), (b), (c) et (d) sur un échantillon de plasma normal témoin ; et
g. à déterminer la différence ou le rapport des temps, des vitesses ou de ces deux paramètres obtenus dans les étapes (e) et (f), ladite différence fournissant une indication du risque de thrombose chez ledit sujet.

2. Méthode pour déterminer le risque de thrombose chez un sujet, ladite méthode comprenant les étapes consistant
a) à ajouter à un échantillon de plasma obtenu chez ledit sujet (i) un premier réactif comprenant un substrat synthétique et (ii) un second réactif qui active la protéine C endogène dans le plasma ;
b) à ajouter à un second échantillon de plasma provenant de ce même sujet (i) un premier réactif comprenant un substrat synthétique et (ii) un tampon ou une autre substance qui n'active pas la protéine C ;
c) à mesurer la vitesse d'hydrolyse du substrat synthétique dans l'échantillon de l'étape (a) ;
d) à mesurer la vitesse d'hydrolyse du substrat synthétique dans l'échantillon de l'étape (b) ;
e) à calculer les différences entre les vitesses obtenues dans les étapes (c) et (d) ;
f) à mettre en oeuvre les étapes (a), (b), (c) et (d) sur un échantillon de plasma normal témoin ; et
g) à déterminer la différence ou le rapport des vitesses obtenues dans les étapes (e) et (f), ladite différence étant une indication du risque de thrombose chez ledit sujet.

3. Méthode suivant la revendication 1, dans laquelle le premier réactif est choisi dans le groupe consistant en le facteur IXa, des réactifs qui engendrent le facteur IXa *in vitro* et le réactif APTT.

4. Méthode suivant la revendication 1, dans laquelle le premier réactif est choisi dans le groupe consistant en le facteur Xa, des réactifs qui engendrent le facteur Xa *in vitro,* des réactifs PT et des activateurs de la voie de coagulation extrinsèque.

5. Méthode suivant la revendication 1 ou 2, dans laquelle le deuxième réactif comprend une fraction purifiée de venin obtenue à partir d'un organisme produisant un venin.

6. Méthode suivant la revendication 5, dans laquelle le venin est un venin de serpent obtenu à partir *d'Agkistrodon contortrix contortrix.*

7. Méthode suivant la revendication 1 ou 2, dans laquelle le deuxième réactif comprend au moins une partie d'une protéine recombinante de venin.

8. Kit pour la détermination du risque de thrombose chez un sujet, ledit kit comprenant :
a. un premier récipient comprenant : (i) un premier réactif en une quantité suffisante pour induire ou activer la coagulation dans un échantillon de plasma obtenu chez ledit sujet, (ii) un deuxième réactif qui active la protéine C endogène dans le plasma et (iii) un troisième réactif comprenant des sels de calcium, des phospholipides ou de la thromboplastine tissulaire ou bien une de leurs associations ;
b. un second récipient pour ajouter à un second échantillon de plasma provenant de ce même sujet (i) ledit premier réactif, (ii) un tampon ou une autre substance qui n'active pas la protéine C, (iii) un troisième réactif comprenant des sels de calcium, des phospholipides ou de la thromboplastine tissulaire ou bien une de leurs associations.

9. Kit pour déterminer le risque de thrombose chez un sujet, ledit kit comprenant :
a) un premier récipient comprenant (i) un premier réactif comprenant un substrat synthétique et (ii) un second réactif qui active la protéine C endogène dans le plasma ;
b) un second récipient pour ajouter à un second échantillon de plasma provenant de ce même sujet (i) ledit premier réactif comprenant un substrat synthétique et (ii) un tampon ou une autre substance qui n'active pas la protéine C.

10. Kit suivant la revendication 8, dans lequel le premier réactif est choisi dans le groupe consistant en le facteur IXa, des réactifs qui engendrent le facteur IXa in *vitro* et des réactifs APTT.

11. Kit suivant la revendication 8, dans lequel le premier réactif est choisi dans le groupe consistant en le facteur Xa, des réactifs qui engendrent le facteur Xa *in vitro,* des réactifs PT et des activateurs de la voie de coagulation extrinsèque.

12. Kit suivant la revendication 8 ou 9, dans lequel le deuxième réactif comprend une fraction purifiée de venin obtenu à partir d'un organisme produisant un venin.

13. Kit suivant la revendication 12, dans lequel le venin est un venin de serpent obtenu à partir *d'Agkistrodon contortrix contortrix.*

14. Kit suivant la revendication 8 ou 9, dans lequel le deuxième réactif comprend une protéine recombinante du venin.
